# EUROPEAN PATENT SPECIFICATION

(11) **EP 2 705 371 B1**
(45) Date of publication and mention of the grant of the patent: **08.07.2015**
(21) Application number: 12731748.5
(22) Date of filing: 02.05.2012
(51) Int. Cl.: G01N 33/574, A61K 39/395

(54) **IDENTIFICATION OF A NEW MOLECULAR FACTOR FOR EARLY DIAGNOSIS OF UROTHELIAL CARCINOMA OF THE BLADDER**
IDENTIFIZIERUNG EINES NEUEN MOLEKULAREN FAKTORS ZUR FRÜHDIAGNOSE VON UROTHELIALEM HARNBLASENKARZINOM
IDENTIFICATION D'UN FACTEUR MOLÉCULAIRE INÉDIT PERMETTANT UN DIAGNOSTIC PRÉCOCE DU CARCINOME UROTHÉLIAL DE LA VESSIE

(30) Priority: 02.05.2011 IT AN20110057
(43) Date of publication of application: 12.03.2014
(73) Proprietor: Lorenzi, Teresa, 60123 Ancona (IT); Lorenzi, Maria, 60123 Ancona (IT); Lorenzi, Stefano, 23871 Lomagna (IT); Zannini, Emanuele, 60035 Jesi (IT)
(72) Inventor: MORRONI, Manrico, I-60121 Ancona (IT)
(74) Representative: Cutropia, Gianluigi
(86) International application number: PCT/IT2012/000128
(87) International publication number: WO 2012/150615

(56) References cited:
- WO-A2-2005/010213
- WO-A2-2009/046405
- DYRSKJÖT L ET AL: "Identifying distinct classes of bladder carcinoma using microarrays", NATURE GENETICS, vol. 33, 2003, pages 90-96, XP002271899,
- SCHULTZ ET AL: "Prediction of recurrence in Ta urothelial cell carcinoma by real-time quantitative PCR analysis: A microarray validation study", INTERNATIONAL JOURNAL OF CANCER, vol. 119, no. 8, 2006, pages 1915-1919, XP002666253,

## Description

### Field of the invention

The present invention relates to the identification of a new early tumoral marker of urothelial carcinoma of the bladder.

### State of the art

In the following description numerals in parenthesis indicate the bibliography listed after the description.

The bladder is the organ where urine filtered by kidneys is collected before being eliminated from the body. Bladder carcinoma consists in the malignant transformation of the cells that compose the bladder; in particular, tumoral cells have characteristics that are morphologically and functionally different from normal cells, replacing the latter. Bladder cancer may diffuse locally and remotely, for example, to lungs, liver and bones through blood circulation. In spite of numerous studies on its genesis and most advanced diagnostic-therapeutic methods, this neoplasia is still characterized by high mortality (1). In fact, bladder carcinoma is one of the most common tumors found by urologists, being the second main cause of death of all neoplasias of the genitourinary tract (1); in men it is the fourth most frequent type after prostatic, pulmonary, and colorectal cancer, accounting approximately for 5-6% of all cancer cases (1). In women it is the eighth most common type of tumor and accounts for approximately 2-3% of all neoplasias (1). Bladder carcinoma is more frequent in the white race and male gender with increment of incidence in advanced age (1, 2). The process that promotes the onset of the neoplasia and its evolution appears to be multi-factorial with involvement of multiple genetic alterations and numerous environmental risk factors. In fact, bladder cancer is the first tumor for which an association with some chemical products has been suggested, in addition to a genetic predisposition that is necessary for the onset and development of the disease. Said chemical products consist in derivatives of benzene and aromatic amines, which are used in the paint industry and, in no lesser extent, nitro-aromatic compounds and nitrosamines identified in the exhaust fumes of diesel engines (3,4,5). Also individuals using hair dyes or smoking cigarettes (6,7,8) are exposed to the risk of bladder cancer.

More than 90% of the bladder malignant neoplasias are represented by carcinomas of urothelial cells, where urothelium indicates the epithelium that lines the excretory system. Such lining keeps on generating new cells through the entire life. However, in some cases, such epithelial growth is uncontrolled and generates tumors. Tumor growth can be superficial when it develops in the vesical lumen (bladder polyp or urinary bladder papilloma) or infiltrating when it develops inside the wall of the bladder (9). Superficial tumors can be treated in a conservative way by means of surgical removal with endoscopic resection (9,10). They usually tend to relapse over time, also after years, and occur in different points of the bladder or excretory system, involving the need for pharmacological therapies and constant endoscopic monitoring (9,10). Only in a small part of the cases (10%) these tumors progressively infiltrate the vesical wall. Infiltrating tumors, which are much more aggressive, require a more demanding therapy, including the complete removal of the bladder (cystectomy) and its reconstruction (9,11). The therapeutic approach involves operations that employ not only surgery, but also radiotherapy, which uses high doses of X-rays to destroy tumoral cells, chemotherapy, which consists in the systemic administration of effective medications to destroy neoplastic cells, even if distant from the site of the primitive tumor, and biological therapy or immunotherapy that aims at stimulating, orientating and restoring the immune system of the organism to fight the tumor through the administration of substances produced by the body or of synthetic origin (12).

An early diagnosis cannot prevent the formation of the tumor, but allows identifying, at an early stage of the disease, the morphological and molecular alterations that indicate the presence of neoplasia. The earlier the cancer is identified, the easier it will be to treat it suitably, and the more will be in general the chances of recovery and survival with the perspective of a longer and qualitatively better life. The advantages of an early diagnosis are considerable also for the community: the treatment of advanced tumoral forms involves very high economic and social costs, with huge difficulties in the organization and management of resources.

At the moment there are no screening programs or early diagnosis methods that are scientifically reliable (12). The collection of clinical data, urine tests searching for blood or tumoral cells and the microscope observation of parts of vesical tissue taken from patients represent today the most standardized and consolidate means to diagnose and monitor bladder cancer (12). However, this type of investigations has a series of limits arising from their heaviness, invasiveness and sometimes poor specificity and sensibility that characterize some of them. Therefore, the aforesaid traditional approaches are not sufficient to recognize and monitor cancer, hence the need to identify biochemical and genetic markers that can be evaluated in biological liquids (serum and urine) (13,14) and also in tissue fragments obtained by means of bioptic analysis (15). In order to permit an early identification of the tumoral onset and progression, said indicators (tumoral markers) must be the mirror of the molecular events that take place in those biological, morphological, and clinical alterations that characterize malignant injuries also in their first stages. The marker is therefore a signal of neoplasia or neoplasia evolution in absence of clinical signs, able to direct more precisely an initial diagnosis or a diagnosis of recovery/relapse (prognosis) from the disease. In order to be defined as such, tumoral markers must be sensible, that is quantifiable in all patients affected by a certain neoplasia, and specific since they are expressed in subjects affected only by such type of neoplasia. They are substances produced directly by the tumor, such as hormones, enzymes or other proteins, more or less related with the numeric growth of tumoral cells, or produced by the organism as a reaction to the tumor, such as proteins of the acute inflammation phase. Tumoral markers that are optimal for clinical routine are accessible with a simple blood or urine sample, which can be repeated over time and measured in any laboratory.

In spite of the decennial efforts made by the scientific community to identify tumoral biomarkers of the bladder, so far it has not been possible to identify an ideal marker, that is a marker that can be easily measured and is characterized by high sensitivity and specificity.

Vice versa, there are numerous substances that can be measured in serum and urine, the simultaneous determination of which provides useful clinical information. Among these substances, first of all, some enzymes must be included, such as thymidylate synthetase, metalloproteinase, hialuronidase and telomerase; some nuclear proteins, such NMP22, some cytokeratins such as tissue polypeptide antigen (TPA), tissue polypeptide-specific (TPS) antigen, urinary bladder cancer (UBC) antigen and Cyfra 21 (9,16).

Together with markers that can be measured in urine and sera, also some tumoral tissue markers have been largely characterized and are currently used in mutual association. They include oncogenes, receptors for the growth factor and membrane antigens revealed with monoclonal antibodies (9,16).

HtrA1 protein (NP_002766.1) (SEQ.1) is a serine protease from the HtrA (High temperature requirement factor A) family, also known as Degp, initially identified in *E.Coli* (17). HtrA1 is a protein that is physiologically present in various tissues, the expression of which is modulated in superior organisms during the development (18) and in pathological situations both of inflammatory (19)-degenerative (20) and neoplastic nature. Numerous studies demonstrate that HtrA1 is downregulated in cancer tissues and produced in higher quantities in normal tissues and that the higher expression inhibits the growth of tumoral cells and their proliferation both in vivo and in vitro (21). Therefore, HtrA1 plays a protective role in many tumoral forms, acting as oncosuppressor (22,23). In line with such a discovery, the mRNA level and the protein expression of HtrA1 significantly reduce with the increasing of the endometrial carcinoma level (24) and the expression of HtrA1 is reduced with the increasing of severity of trophoblastic disease (25). HtrA1 acts on neoplastic cells also as endogen mediator of the chemotherapeutic action of cisplatin. In fact, HtrA1 levels can be considered as predictive of the tumoral response against the treatment with cisplatin both of ovarian and stomach tumors (26). The expression of HtrA1 has been recently analyzed in a large group of mesotheliomas and the study has shown that its expression is significantly and positively related with survival of patients (27).

The well-known biological implications of HtrA1 in the development of normal tissues, as well as in the progression of some neoplasias, have suggested us to investigate the presence, the possible alteration and role of HtrA1 in bladder urothelial carcinoma.

WO2005/010213 discloses a kit comprising antibodies of the HrtA1 protein

WO2009/046405 discloses a pharmaceutical composition comprising cytotoxic antibodies of the HrtA1 protein.

The "INTERNATIONAL JOURNAL OF CANCER" VOL 119, (NO. 8 2006, Pages 1915-1919) scientific publication discloses a biomarker for the diagnosis of bladder cancer.

### Description of the invention

In its broadest sense the invention is defined by the following items.
1) Method for the early diagnosis of urothelial carcinoma of the bladder comprising the use of a biomarker comprising HtrA1 protein and the determination of the amount of HtrA1 protein, in samples from any examinee.
2) Method of item 1, wherein HtrA1 protein is constituted:
   - by the amino acid sequence NP_002766.1 (SEQ. 1) or
   - by an auto-proteolytic fragment consisting of 38 kDa form or
   - by variants of HtrA1 protein having an amino acid sequence sharing at least 80% amino acid identity to sequence SEQ. 1 or to the sequence of the respective auto-proteolytic fragment consisting of 38 kDa form, or
   - by a molecule that encompasses the amino acid sequence SEQ. 1.
3) Method according to item 2, wherein the amino acid sequence SEQ. 1 or its respective variants, or the protein molecule that encompasses the amino acid sequence SEQ. 1 are immunoprecipitated from suitably concentrated urine according to a standard method.
4) Method of item 3, wherein concentrations in urine are normalized on the basis of creatinine concentration.
5) Method according to item 2, wherein the auto-proteolytic fragment of the amino acid sequence SEQ. 1 or its variants are analyzed directly in protein extracts from bladder tissues.
6) Method of item 5, wherein bladder protein extracts are normalized on the basis of the value of β-actin used as the housekeeping protein.
7) Method of any one of the preceding items, wherein said samples are represented by the examinee's biological fluids or bladder tissue.
8) Method of item 7, wherein said biological fluids include urine.
9) Method according to any one of the preceding items, wherein the amount of HtrA1 protein, measured in the examinee's samples, is compared to the amount determined in subjects considered healthy, and wherein a difference in said amount between the two samples is associated with the presence of urothelial carcinoma of the bladder.
10) Method according to any one of items 2 to 8, wherein an increased amount of HtrA1 protein SEQ. 1 or of its variants, or of the protein molecule encompassing the amino acid sequence SEQ. 1, in the examinee's urine compared with urine of subjects considered healthy, is indicative of the presence of urothelial carcinoma of the bladder.
11) Method according to any one of items 2 to 8, wherein a reduced amount of the auto-proteolytic fragment of the amino acid sequence SEQ. 1 or of its variants, according to items 2, 5 and 6, in the examinee's tissue compared with tissue of subjects considered healthy, indicates urothelial carcinoma of the bladder.
12) Method to monitor treatment effectiveness of urothelial carcinoma of the bladder, wherein HtrA1 protein is determined, according to a method of items 10 and 11, in urine or tissue from patients.
13) Method for bladder cancer imaging, wherein the patient receives an antibody that binds specifically to HtrA1 protein SEQ. 1 or to the auto-proteolyc fragment or to the respective variants, or to the molecule that encompasses the amino acid sequence SEQ. 1, according to item 2.
14) Method of item 13, wherein the antibody is conjugated to a detectable marker.

The immunohistochemistry studies carried out by the applicant demonstrate that HtrA1 is a molecule expressed by the urothelium of the bladder in physiological conditions and in inflammatory pathologies, such as bacterial cystitis. On the contrary, the protein is absent at urothelial level in all examined cases of urothelial carcinoma with different malignity level and at different infiltration stages, hence from the earliest stages.

Moreover, the Western Blotting showed two bands, one of 38 kDa and one of 53 kDa, in normal and neoplastic vesical tissues. The 38 kDa form is probably originated from the autocatalysis of the native 53 kDa form, as described in literature (28). With reference to the 53 kDa band, no expression difference was noted between the physiological condition and the pathological condition, whereas the 38 kDa band always showed a reduction in pathological tissues. These results suggested that HtrA1, and in particular its form with lower molecular weight, may be a good candidate to be considered a new early tumoral marker of the urothelial bladder carcinoma.

Since this protein was originally described as a secretion protease (29), our data induced us to think that it may be secreted by the urothelium in the bladder cavity and be therefore traced at urinary level, being easy to measure. During the second stage of our study we examined the presence of HtrA1 in the urine of healthy subjects, in the urine of patients with urothelial carcinoma with different malignity level and at different infiltration stages and in subjects affected by bacterial cystitis. Just like for tissues, the Western Blotting carried out on urine samples showed two bands: one of 38 kDa and one of 53 kDa. The 53 kDa form proved to be considerably and significantly more represented in pathological urine compared to the urine of healthy subjects and subjects with cystitis, being prevalently negative. Instead, the 38 kDa form in urine proved to be not homogeneously present in pathological conditions and mainly absent in physiological conditions and in inflammatory state.

Therefore, the present invention relates to the identification of the two forms of HtrA1 protein, the 38 kDa form in bladder tissue and the 53 kDa form in urine, as useful tumoral markers for the early diagnosis of urothelial bladder carcinoma.

Additional characteristics of the invention will appear clearer from the following detailed description, with reference to the attached drawings, wherein:

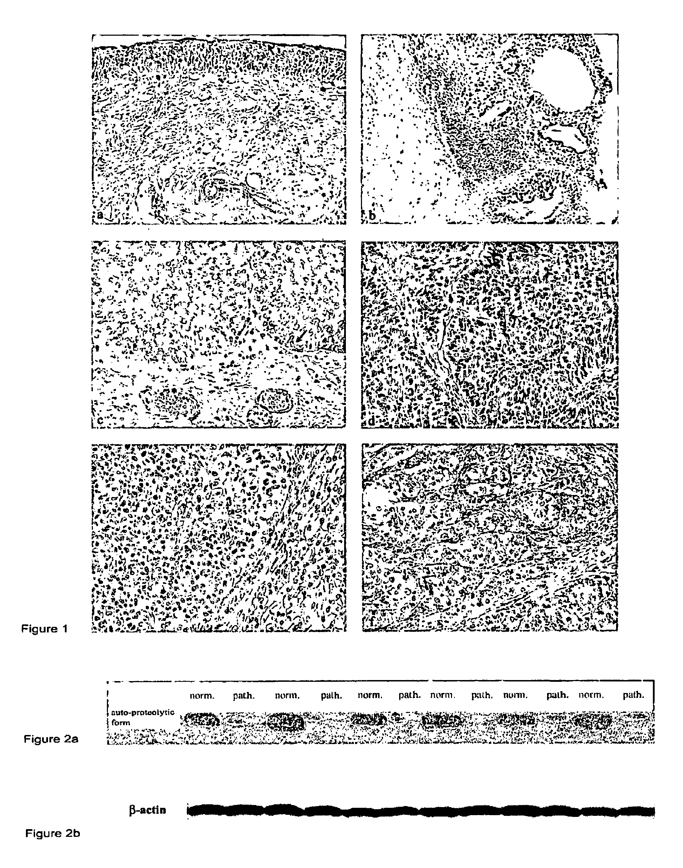

Fig. 1 represents the immunohistochemical reaction of HtrA1 in samples of normal tissue, tumoral tissue and tissue with bacterial cystitis. a) Normal bladder tissue. b) Cystitis. The arrow indicates the higher positivity in the apical area of the cupoliform cells. c) papilliferous neoplastic urothelium with low malignity potential: the arrow indicates the cells of the basal urothelial layer that are still positive, although weakly, for HtrA1. The asterisk in a) and c) indicates the expression of HtrA1 in the tunica media of stromal vessels; d, e) papilliferous neoplastic urothelium with growing malignity potential; f) neoplastic infiltrating urothelial tissue.
Fig. 2a is a representative image of the protein expression of the auto-proteolytic form (38 kDa) of HtrA1 in pairs of normal bladder tissue (norm.) and pathological (pat.) bladder tissue.
Fig. 2b is a representative image of the protein expression of β-actin (b) in pairs of normal bladder tissue (norm.) and pathological (pat.) bladder tissue.
Fig. 3 is a semiquantitative analysis of the auto-proteolytic form (38 kDa) of HtrA1 in normal and pathological bladder tissues.
Fig. 4 is a representative image of the protein expression of the native form (53 kDa) of HtrA1 in urine samples of patients with urothelial carcinoma who underwent radical cystectomy (r. cyst.) or TURB.
Fig. 5 is a representative image of the protein expression of the native form (53 kDa) of HtrA1 in urine samples of healthy subjects and subjects with cystitis.
Fig. 6 is a semiquantitative analysis of the native form (53 kDa) of HtrA1 in all of the analyzed urine cases.

The procedure followed to achieve the aforesaid results is described hereinafter in detail.

### STEP 1 - Collection of tissue and urine samples to be tested.

Tissue samples of urothelial carcinoma with different malignity level (2004 WHO classification) (30) and at different infiltration stages (2002 TNM classification) (31) were collected. Invasive tumoral tissues and visually normal tissue samples of the same bladder, far from the area affected by the pathology, were obtained from patients who underwent radical cystectomy (n=18), (Table 1 and 2 below).

**Table 1**

| | |
|---|---|
| **Number of patients** | **58** |
| **Average age** | **68.2** |
| **Men/women ratio** | **2 ÷ 1** |
| **Bacterial cystitis** | **10** |
| **Papilliferous urothelial neoplasia with low malignity potential*** | **10** |
| **Papilliferous low**-**level non-invasive carcinoma*** | **10** |
| **Papilliferous high-level non-invasive carcinoma*** | **10** |
| **Invasive urothelial carcinoma**** | **18** |
| | |
| | **Stage pT1 (3)** |
| | **Stage pT2a (3)** |
| | **Stage PT2b (3)** |
| | **Stage pT3a (3)** |
| | **Stage pT3b (3)** |
| | **Stage pT4 (3)** |

| | |
|---|---|
| **(*) Grading according to the WHO classification (30)** **(**) Staging according to the TNM classification (31)** | |

**Table 2 Complete case histories of tissue and urine samples tested in immunohistochemistry and in Western Blotting.**

| | | **Sample Type** | | |
|---|---|---|---|---|
| | | **Tissue** | | **Urine** |
| | | **Analysis techniques** | | |
| **Sample origin** | **Total number of cases** | **IIC*** | **WB**** | |
| **TURB** | 30 | yes† | no^{§} | yes |
| **Radical cystectomy** | 18 | yes | yes | yes |
| **Bacterial cystities (biopsies)** | 10 | yes | no | yes |
| **Healthy subjects** | 15 | no | no | yes |

| | | | | |
|---|---|---|---|---|
| * Immunohistochemistry ** Western Blotting ^{§} Analysis not carried out ^{†} Analysis carried out | | | | |

Moreover, samples of papilliferous tumoral tissue were taken with vesical biopsy from patients who underwent TURB (n=30), as well as samples of inflamed tissue from subjects affected by bacterial cystitis (n=10) (Tables 1, 2). Patients affected by urothelial carcinoma and cystitis (68.2 average age) also provided the urine samples used in this study. Moreover, urine samples from healthy subjects (n=15) (62 average age) were analyzed (Table 2). All patients, admitted at the Urological Clinic of the Polytechnic University of Marche, signed the informed consent and were not affected by any other pathology.

### STEP 2 - Immunohistochemistry of samples of normal bladder tissue, tumoral bladder tissue and tissue from subjects affected by cystitis.

For the immunohistochemical analysis (Table 2), the samples of normal bladder tissue, tumoral bladder tissue and tissue from subjects affected by cystitis were fixed in formalin at 4%, included in paraffin and cut in 3µm sections.

The paraffin sections were deparaffined and rehydrated through passages in xylene and in a graduated series of alcohols with decreasing percentage (from 100% to 50%), incubated for one hour with hydrogen peroxide at 3% in deionized water to inhibit the activity of endogenous peroxidase and finally treated for 10 minutes at 37°C with 0.1% trypsin and 0.1% calcium chloride in Tris-HCl 0.05M pH 7.6. Then the sections were washed in Tris-HCl. To eliminate the possible background due to non specific bonds, the sections were incubated for one hour at room temperature and under agitation in non-fat dry milk (NFDM) (BioRad, Hercules CA) at 6% in Tris-HCl. Overnight incubation followed at 4°C with primary polyclonal antibody prepared in rabbit against HtrA1 human protein (Abcam, Cambridge, UK), diluted 1:100 in Tris-HCl+ NFDM 3%. After some washings in Tris-HCl, the sections were incubated for one hour with biotinylated anti-rabbit secondary antibody diluted 1:200 in Tris-HCl+NFDM 3%. After some additional washings in Tris-HCl, the immunohistochemical reaction was detected with the ABC method (Vector Laboratories, Burlingame, CA), (1 h at room temperature) using DAB (3'3'diaminobenzidine tetrahydrochloride) (SIGMA-ALDRICH, Milano, Italia) as chromogen. After being washed with tap water and then with deionized water, the sections were counterstained with Mayer's haematoxylin (Merck Chemicals SpA, Milano, Italia) for approximately 2 minutes, placed under running water and dehydrated. Then slides were mounted using the Eukitt solution (Kindler GmbH and Co., Freiburg, Germany).

The normal bladder samples and the samples of subjects affected by cystitis showed immunostaining for HtrA1 prevalently homogeneous at urothelial level, present in all layers of the urothelium, but with higher positivity in the apical area of the cupoliform cells (Figure 1a,b). The tunica media of the vesical vessels, as well as the muscular tunica of the bladder, were positive for HtrA1 (Figure 1a).

In the urothelium of papilliferous neoplasias with low malignity potential, HtrA1 proved to be weakly present in the cells of the basal urothelial layer (Figure 1c), whereas the higher urothelial layers proved to be negative for HtrA1. Instead, positivity was still observed in the tunica media of stromal vessels, as well as in the muscular tunica of the bladder. The other neoplasias that were examined, meaning papilliferous neoplasias with growing malignity potential and infiltrating neoplasias, showed prevalently negative immunostaining for HtrA1 at urothelial cell level, whereas the muscular tunica of the bladder and the tunica media of the vessels showed weak positivity for HtrA1 (Figures 1d,e,f).

### STEP 3 - Preparation of protein extracts of normal and invasive tumoral bladder tissue samples for Western Blotting analysis

Portions of normal and tumoral bladder tissue, adjacent to the ones analyzed in immunohistochemistry, were immediately frozen in liquid nitrogen after the surgical sampling and then stored at -80°C. The tumoral tissue samples only came from bladders of patients who underwent radical cystectomy because with TURB the recovered tissue material was insufficient for the Western Blotting analysis. For the same reason, also the tissues taken with bladder biopsy from subjects affected by cystitis were not examined with such technique (Table 2).

Before being analyzed, the bladder tissue samples were defrosted and washed with 0.1 M pH 7.4 sodium phosphate buffer. At every washing, in order to avoid losing material, the samples were centrifuged at 800g for 1 minute at 4°C. Then, they were weighed and transformed in powder by means of manual breaking with pestle and mortar. Every sample was resuspended in lysis buffer (Tris-HCl 20mM, pH 8.0, Nonidet-P40 1%, glycerol 10%, NaCl 137mM, CaCl₂ 1mM, MgCl₂ 1mM) containing protease inhibitors (SIGMA-ALDRICH), setting the 0.1g tissue/1ml buffer ratio. Then, the samples were homogenized with potter (Ultra-Turrax T8, IKA®-WERKE, Lille, France) and centrifuged at 20000g. The supernatants that were obtained, defined as raw extracts, were aliquoted and stored at -80°C. Protein concentration was measured with the Bradford method (32).

### STEP 4 - Analysis in Western Blotting of samples of normal bladder tissue and invasive tumoral bladder tissue.

The protein extracts to be analyzed to quantitize the band relevant to the autopreolytic product of HtrA1 were prepared by denaturing 100µg of total proteins with sample buffer 1X (Tris-HCl 1M pH 6.8, SDS 2% e β-mercaptoethanol 1%), keeping them boiling for 8 minutes.

Then, the samples were subjected to denaturing electrophoretic run in SDS-PAGE (sodium dodecyl sulfate-polyacrylamide gel electrophoresis) with Laemmli method (33), making a constant current of 14mA pass for 20 hours in a 10% acrylamide gel. After terminating the electrophoretic run, proteins were transferred from the gel to a polyvynilidene fluoride (PVDF) membrane, using the Tris-base 20mM, glycine 150mM, methanol 20% buffer. The transfer was continued for one night, at constant voltage (30V) at 4°C. Then the membrane was washed with water, then in methanol for 15-20 seconds to fix the bands, then in water again, and finally in TBS-T (Tris-HCl 0.02M, pH 7.5, NaCl 0,15M, Tween-20 0.1%). Successively, it was immersed for 2 hours in agitation in the blocking buffer (TBS-T + BSA 5%) in order to increase the specificity of the antibody to its target protein. Then the membrane was incubated for the night at 4°C with monoclonal primary antibody directed against the HtrA1/PRSS11 human protein (amino acids 23-480) (R&D Systems, Minneapolis, USA) diluted 1:1000 in TBS-T + BSA 5%. After 8 washings of 8 minutes each with TBS-T, the membrane was incubated for 2 hours at room temperature with anti IgG murine secondary antibody conjugated to horseradish peroxidase (HRP) (Amersham Srl, Milano Italia), diluted 1:5000 in TBS-T + BSA 5%, and then washed again for 8 times in one hour with TBS-T. To display the β-actin band, a protein used as housekeeping for data normalization, the primary monoclonal anti-β-actin antibody (SIGMA) was used and incubated for the night at 4°C.

For the development of the chemoluminescence reaction, the ECL - plus solution (Amersham Srl) was used, acting on the membrane for 5 minutes. The membrane was dried and then coated with film, inserted in an autoradiographic case together with the plate and exposed overnight. The development and fixation were carried out.

Figure 2a shows the Western Blotting image representative of the protein expression of the autoproteolytic form of HtrA1 in bladder tissue samples, which shows a significant constant difference in expression between normal and pathological samples. In particular, the autoproteolytic form of HtrA1 was significantly reduced in pathological tissue samples, where it appears extremely weak and difficult to detect in some cases.

The protein expression in Western Blotting of β-actin in the same lysates of normal and pathological bladder tissues already analyzed to evaluate the levels of the autoproteolytic form of HtrA1 confirmed the gel homogenous loading (Figure 2b).

### STEP 5 - Calculation of molecular weight, densitometric analysis and statistical evaluation of signals, obtained in Western Blotting, of the auto-proteolytic form of HtrA1 in normal and invasive tumoral bladder tissue samples.

The equation and correlation coefficient R² were obtained from the calibration line constructed by considering the molecular weights (P.M.) of each standard protein and the corresponding Rf (relative mobility).

Inserting the Rf measure of the band relevant to the autoproteolytic form of HtrA1 in the equation of the calibration line, the molecular weight was calculated, being 38 kDa, as mentioned in literature (28).

The images of the autoradiographic plates were acquired with a scanner and transformed in TIFF format, eliminating any information on color, and then analyzed in optical densitometry with Quantity One 4.6.1 (BioRad) program. The quantity of protein relative to the band of HtrA1 at 38 kDa was expressed in intensity values (in grey scale).

β-actin was used as loading control and its expression values, obtained from the densitometric analysis, were used to normalize the densitometric data relevant to the expression of the autoproteolityc form of HtrA1.

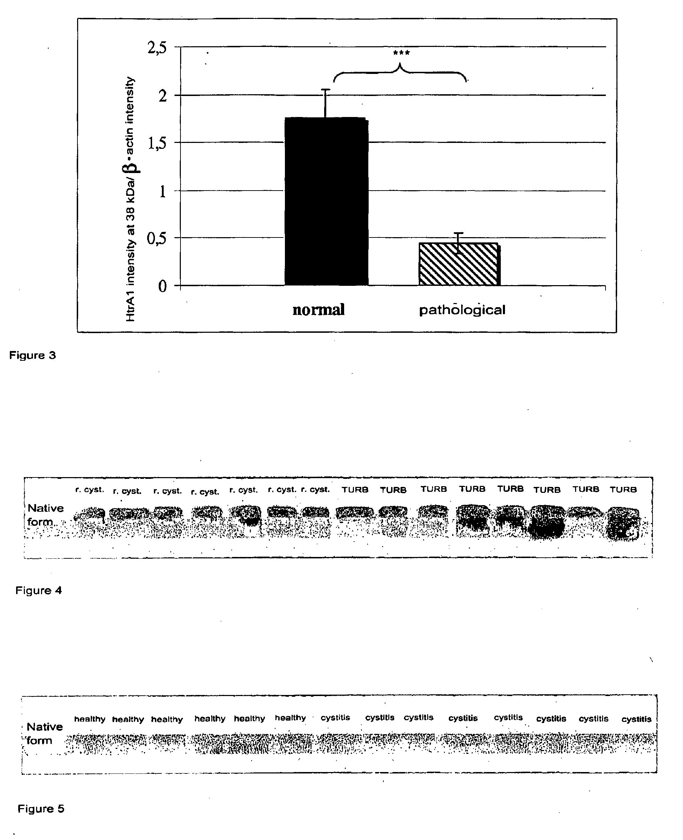

The results were presented as mean ± standard deviation (SD). The statistical analysis of data was performed using the two-tailed T-test. From the semi-quantitative analysis of HtrA1, we observed an expression reduction of the 38 kDa form in pathological tissues compared to normal tissues and such a reduction proved to be highly significant (p<0.001) (***) (Figure 3).

### STEP 6 - Preparation of urine samples of healthy subjects, subjects with cystitis and patients affected by urothelial carcinoma for Western Blotting analysis.

Urine samples of healthy subjects, of subjects with cystitis and of patients affected by urothelial carcinoma (Tables 1, 2) were collected in sterile containers at the first morning urination and, in case of carcinoma, taken the same day of the surgical operation (TURB or radical cystectomy). Immediately centrifuged for 15 minutes at 1500g at 4°C, they originated a pellet and a supernatant, the latter being stored at -20°C.

Upon the analysis, urine was defrosted, centrifuged for 20 minutes at 3000g at 4°C and taken to the Analysis Laboratory of Azienda Ospedaliera Universitaria Ospedali Riuniti "Umberto I -G.M. Lancisi -G. Salesi (Ancona) to measure creatinine (mg/dl) and protein concentration (g/L).

The urine samples were then concentrated with suitable devices (Amicon Ultra-4 and Amicon Ultra-15) (Millipore, Milan, Italy), choosing as reference a normal urine sample that, being previously concentrated and analyzed in Western Blotting, showed a quantizable signal. Then, all urine samples were concentrated for a variable number of times according to the relevant creatinine values related with the value of the reference sample (normalization). Then concentrates were stored at -20°C.

### STEP 7 - Analysis in Western Blotting of urine samples of healthy subjects, subjects with cystitis and patients affected by urothelial carcinoma.

The immunoprecipitation method was used for quantization of the native form of HtrA1 in urine. We used a 50% suspension of sepharose spheres bound to G protein (GE Healthcare, Bio-Sciences AB, Uppsala, Sweden) equilibrated in lysis buffer. For each urine sample to be immunoprecipitated, 50µl of the sphere suspension were incubated for the night, at 4°C and in rotation, with 3µg of the monoclonal primary antibody directed against HtrA1/PRSS11 human protein (amino acids 23-480) (R&D Systems). The sepharose spheres were first washed for 8 times with the lysis buffer, then, once collected, they were delicately resuspended in 50µl of the same buffer and finally incubated for the night at 4°C and in rotation with 225µl of each concentrated urine. Then, they were collected, washed for 3 times with the lysis buffer and centrifuged at 13000g for 1 minute for pelleting. The bound proteins were eluted from the spheres through incubation with 45µl of glycine 100mM, pH 2.5 for 30 minutes at room temperature and in agitation. Elutes (40µl) were then collected, denatured in sample buffer 1X, neutralized with soda and boiled for 8 minutes.

Urine immunoprecipitates were loaded in a 10% acrylamide gel and transferred on PVDF membrane. Then, antibody reaction was carried out with monoclonal primary antibody directed against the HtrA1/PRSS11 human protein (amino acids 23-480) (R&D Systems), and detection of bands was performed by means of chemoluminescence, following the same protocol used for tissues.

The expression of urinary HtrA1 in its native form (53 kDa) was positive, although in a variable way, in 100% of the patients with urothelial carcinoma who underwent radical cystectomy or TURB (Figure 4), whereas was practically absent in all normal cases and in subjects with cystitis (Figure 5), thus suggesting that HtrA1 is not generally involved in inflammatory pathologies.

### STEP 8 - Densitometric analysis and statistical evaluation of signals, obtained in Western Blotting, of the native form of HtrA1 in urine samples of healthy subjects, subjects with cystitis and patients affected by urothelial carcinoma.

By means of densitometry, the intensity values (in grey scale) of the native form of HtrA1 were calculated in all urine samples that were analyzed. The values were submitted to the one-way variance analysis (ANOVA).

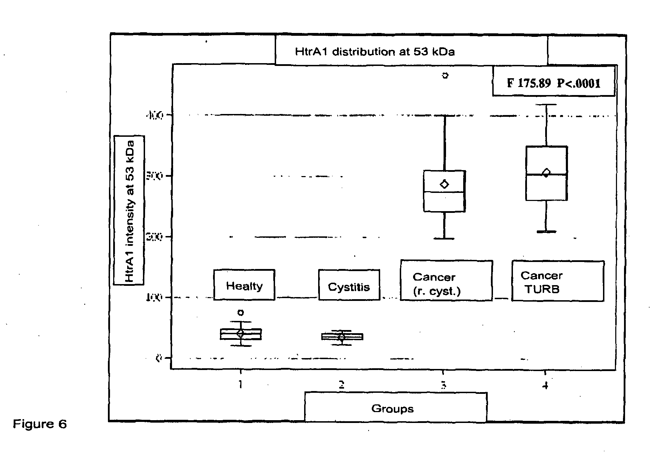

From the semi-quantitative analysis of HtrA1, we observed a significant expression increment of the 53 kDa form in the urine of patients with urothelial carcinoma, who underwent total cystectomy and TURB, compared to urine of healthy subjects or subjects with cystitis (Figure 6). These increments proved to be highly significant (p<0.0001).

### Bibliography

1) Jemal A, Siegel R, Xu J, et al. Cancer statistics, 2010. CA Cancer J Clin. 2011;61(2):133-4.
2) Horner MJ, Ries LA, Krapcho M, et al, eds. SEER Cancer Statistics Review, 1975-2006. Bethesda, MD: National Cancer Institute; 2009.
3) Vineis P, Pirastu R. Aromatic amines and cancer. Cancer Causes Control. 1997;8:346-55.
4) Delclos GL, Lerner SP. Occupational risk factors. Scand J Urol Nephrol Suppl. 2008;(218):58-63.
5) Nilsson S, Ullen A. Chemotherapy-induced bladder cancer. Scand J Urol Nephrol Suppl. 2008;(218):89-92.
6) Takkouche B, Etminan M, Montes-Martinez A. Personal use of hair dyes and risk of cancer; a meta-analysis JAMA 2005;293(20):2516-25.
7) Brennan P, Bogillot O, Cordier S, et al. Cigarette smoking and bladder cancer in men: a pooled analysis of 11 case-control studies. Int J Cancer. 2000;86(2):289-94.
8) Brennan P, Bogillot O, Greiser E, et al. The contribution of cigarette smoking to bladder cancer in women (pooled European data). Cancer Causes Control. 2001 ;12(5):411-7.
9) Jacobs BL, Lee CT, Montie JE. Bladder cancer in 2010: how far have we come? CA Cancer J Clin. 2010;60(4):244-72. Review.
10) Nieder AM, Brausi M, Lamm D, et al. Management of stage T1 tumors of the bladder: International Consensus Panel. Urology. 2005;66:108-125.
11) Stein JP, Lieskovsky G, Cote R, et al. Radical cystectomy in the treatment of invasive bladder cancer: long-term results in 1,054 patients. J Clin Oncol. 2001;19:666-675.
12) Pashos CL, Botteman MF, Laskin BL, et al. Bladder cancer: epidemiology, diagnosis, and management. Cancer Pract. 2002;10(6):311-22. Review.
13) Minami S, Sato Y, Matsumoto T, et al. Proteomic study of sera from patients with bladder cancer: usefulness of S100A8 and S100A9 proteins. Cancer Genomics Proteomics. 2010;7(4):181-9.
14) Dey P. Urinary markers of bladder carcinoma. Clin Chim Acta. 2004;340(1-2):57-65. Review.
15) Van QN, Veenstra TD, Issaq HJ. Metabolic Profiling for the Detection of Bladder Cancer. Curr Urol Rep. 2011;12(1):34-40.
16) Cheng L, Zhang S, Maclennan GT, et al. Bladder cancer: translating molecular genetic insights into clinical practice. Hum Pathol. 2011;42(4):455-81.
17) Pallen MJ, Wren BW. The HtrA family of serine proteases. Mol Microbiol. 1997;26(2):209-21. Review.
18) De Luca A, De Falco M, Severino A, et al. Distribution of the serine protease HtrA1 in normal human tissues. J Histochem Cytochem. 2003;51(10):1279-84.
19) Grau S, Richards PJ, Kerr B, et al. The role of human HtrA1 in arthritic disease. J Biol Chem. 2006;281(10):6124-9.
20) Yang Z, Camp NJ, Sun H, et al. A variant of the HTRA1 gene increases susceptibility to age-related macular degeneration. Science. 2006;314(5801):992-3.
21) Baldi A, De Luca A, Morini M, et al. The HtrA1 serine protease is down-regulated during human melanoma progression and represses growth of metastatic melanoma cells. Oncogene. 2002;21(43):6684-8.
22) Shridhar V, Sen A, Chien J, et al. Identification of underexpressed genes in early- and late-stage primary ovarian tumors by suppression subtraction hybridization. Cancer Res. 2002;62(1):262-70.
23) Chien J, Staub J, Hu SI, et al. A candidate tumor suppressor HtrA1 is downregulated in ovarian cancer. Oncogene. 2004;23(8):1636-44.
24) Bowden MA, Di Nezza-Cossens LA, Jobling T, et al. Serine proteases HTRA1 and HTRA3 are down-regulated with increasing grades of human endometrial cancer. Gynecol Oncol. 2006;103(1):253-60.
25) Marzioni D, Quaranta A, Lorenzi T, et al. Expression pattern alterations of the serine protease HtrA1 in normal human placental tissues and in gestational trophoblastic diseases. Histol Histopathol. 2009;24(10):1213-22.
26) Chien J, Aletti G, Baldi A, et al. Serine protease HtrA1 modulates chemotherapy-induced cytotoxicity. J Clin Invest. 2006;116(7):1994-2004.
27) Baldi A, Mottolese M, Vincenzi B, et al. The serine protease HtrA1 is a novel prognostic factor for human mesothelioma. Pharmacogenomics. 2008;9(8):1069-77.
28) Tocharus J, Tsuchiya A, Kajikawa M, et al. Developmentally regulated expression of mouse HtrA3 and its role as an inhibitor of TGF-beta signaling. Dev Growth Differ. 2004;46(3):257-74.
29) Clausen T, Southan C, Ehrmann M. The HtrA family of proteases: implications for protein composition and cell fate. Mol Cell. 2002 Sep;10(3):443-55. Review.
30) Sauter G, Algaba F, Amin MB, Busch C, Cheville J, Gasser T, Grignon D, Hofstädter F, Lopez-Beltran A, Epstein JI. Non-invasive urothelial tumours. In World Health Organization Classification of Tumours, Pathology and Genetics: Tumours of the Urinary System and Male Genital Organs. Edited by JN Eble, G Sauter, JI Epstein, IA Sesterhenn. IARC Press, Lyon, France, 2004, pp. 110-23.
31) Sobin LH and Wittekind C. TNM classification of malignant tumours. Sixth Edition, Sobin LH and Wittekind C (eds). Wiley-Liss: New York, 2002.
32) Bradford MM. A rapid and sensitive method for the quantitation of microgram quantities of protein utilizing the principle of protein-dye binding. Anal Biochem. 1976;72:248-54.
33) Laemmli UK. Cleavage of structural proteins during the assembly of the head of bacteriophage T4. Nature. 1970;227(5259):680-5.

## Claims

1. Method for the early diagnosis of urothelial carcinoma of the bladder comprising the use of a biomarker comprising HtrA1 protein and the determination of the amount of HtrA1 protein, in samples from any examinee.

2. Method of claim 1, wherein HtrA1 protein is constituted:
- by the amino acid sequence NP_002766.1 (SEQ.1) or
- by an auto-proteolytic fragment consisting of 38 kDa form or
- by variants of HtrA1 protein having an amino acid sequence sharing at least 80% amino acid identity to sequence SEQ. 1 or to the sequence of the respective auto-proteolytic fragment consisting of 38 kDa form, or
- by a molecule that encompasses the amino acid sequence SEQ. 1.

3. Method according to claims 2, wherein the amino acid sequence SEQ. 1 or its respective variants, or the protein molecule that encompasses the amino acid sequence SEQ. 1 are immunoprecipitated from suitably concentrated urine according to a standard method.

4. Method of claim 3, wherein concentrations in urine are normalized on the basis of creatinine concentration.

5. Method according to claim 2, wherein the auto-proteolytic fragment of the amino acid sequence SEQ. 1 or its variants are analyzed directly in protein extracts from bladder tissues.

6. Method of claim 5, wherein bladder protein extracts are normalized on the basis of the value of β-actin used as the housekeeping protein.

7. Method of any one of the preceding claims, wherein said samples are represented by the examinee's biological fluids or bladder tissue.

8. Method of claim 7, wherein said biological fluids include urine.

9. Method according to any one of the preceding claims, wherein the amount of HtrA1 protein, measured in the examinee's samples, is compared to the amount determined in subjects considered healthy, and wherein a difference in said amount between the two samples is associated with the presence of urothelial carcinoma of the bladder.

10. Method according to any one of claims 2 to 8, wherein an increased amount of HtrA1 protein SEQ. 1 or of its variants, or of the protein molecule encompassing the amino acid sequence SEQ. 1, in the examinee's urine compared with urine of subjects considered healthy, is indicative of the presence of urothelial carcinoma of the bladder.

11. Method according to any one of claims 2 to 8, wherein a reduced amount of the auto-proteolytic fragment of the amino acid sequence SEQ. 1 or of its variants, according to claims 2, 5 and 6, in the examinee's tissue compared with tissue of subjects considered healthy, indicates urothelial carcinoma of the bladder.

12. Method to monitor treatment effectiveness of urothelial carcinoma of the bladder, wherein HtrA1 protein is determined, according to a method of claims 10 and 11, in urine or tissue from patients.

13. Method for bladder cancer imaging, wherein the patient receives an antibody that binds specifically to HtrA1 protein SEQ. 1 or to the auto-proteolyc fragment or to the respective variants, or to the molecule that encompasses the amino acid sequence SEQ. 1, according to claim 2.

14. Method of claim 13, wherein the antibody is conjugated to a detectable marker.

## Patentansprüche

1. Verfahren zur Frühdiagnose von Urothelkarzinomen in der Blase, das die Verwendung eines Biomarkers vorsieht, umfassend das HtrA1-Protein und die Bestimmung der HtrA1-Proteinmenge in Stichproben einer beliebigen Testperson.

2. Verfahren nach Anspruch 1, bei dem
- das HtrA1-Protein aus Folgendem besteht:
- aus der Aminosäurensequenz NP_002766.1 (SEQ. 1) oder
- aus einem autoproteolytischen Fragment in 38kDa-Form oder
- aus Varianten des HtrA1-Proteins mit einer Aminosäurensequenz, die zu 80% identisch mit der Aminosäurensequenz SEQ. 1 oder mit der Sequenz des entsprechenden autoproteolytischen Fragments ist, das aus der 38kDa-Form besteht, oder
- aus einem Molekül, das die Aminosäurensequenz SEQ 1 umfasst.

3. Verfahren nach Anspruch 2, bei dem die Aminosäurensequenz SEQ. 1 oder deren entsprechende Varianten oder das Proteinmolekül, das die Aminosäurensequenz SEQ. 1 umfasst, aus entsprechend konzentriertem Urin mittels eines Standardverfahrens immunpräzipiert sind.

4. Verfahren nach Anspruch 3, bei dem die Urinkonzentrationen auf der Basis der Kreatinkonzentration normalisiert sind.

5. Verfahren nach Anspruch 2, bei dem das autoproteolytische Fragment der Aminosäurensequenz SEQ. 1 oder deren Varianten direkt in den Proteinextrakten aus Blasengewebe analysiert werden.

6. Verfahren nach Anspruch 5, bei dem die Normalisierung der Proteinextrakte aus Blasengewebe auf der Basis des Wertes von β-Actin vorgenommen wird, das als Housekeeping-Protein verwendet wird.

7. Verfahren nach einem beliebigen der vorstehenden Ansprüche, bei dem die Stichproben aus den biologischen Flüssigkeiten oder dem Blasengewebe der Testperson bestehen.

8. Verfahren nach Anspruch 5, bei dem die biologischen Flüssigkeiten Urin umfassen.

9. Verfahren nach einem beliebigen der vorstehenden Ansprüche, bei dem die Menge des HtrA1-Proteins, das in den Stichproben der Testperson gemessen wird, mit der Menge verglichen wird, die bei einer als gesund eingestuften Person festgestellt wird, und bei dem ein Mengenunterschied zwischen den beiden Stichproben mit dem Vorliegen eines Blasenkarzinoms assoziiert wird.

10. Verfahren nach einem der Ansprüche von 2 bis 8, bei dem eine erhöhte Menge des Proteins HtrA1 SEQ. 1 oder dessen Varianten oder des Proteinmoleküls, das die Aminosäurensequenz SEQ. 1 umfasst, im Urin der Testperson im Vergleich zu der als gesund eingestuften Person als Hinweis auf ein Urothelkarzinom der Blase gewertet wird.

11. Verfahren nach einem der Ansprüche von 2 bis 8, bei dem eine verminderte Menge des autoproteolytischen Fragments der Aminosäurensequenz SEQ 1 oder deren Varianten nach den Ansprüchen 2, 5 und 6 im Gewebe der Testperson im Vergleich zu der als gesund eingestuften Person als Hinweis auf ein Urothelkarzinom der Blase gewertet wird.

12. Verfahren zur Überwachung der Wirksamkeit der Behandlung von Urothelkarzinomen der Blase, bei dem das HtrA1-Protein nach dem Verfahren gemäß den Ansprüchen 10 und 11 im Urin oder im Gewebe eines Patienten quantifiziert wird.

13. Verfahren zur Bildgebung von Blasenkrebs, bei dem dem Patienten ein Antikörper verabreicht wird, der spezifisch an das Protein HtrA1 SEQ 1 oder an dessen autoproteolytisches Fragment oder an dessen entsprechende Varianten oder an das Proteinmolekül bindet, das die Aminosäurensequenz SEQ. 1 nach Anspruch 2 umfasst.

14. Verfahren nach Anspruch 13, bei dem der Antikörper an einen nachweisbaren Marker konjugiert ist.

## Revendications

1. Méthode pour le diagnostic précoce du cancer urothélial de la vessie qui prévoit l'utilisation d'un biomarqueur comprenant la protéine HtrA1 et la détermination de la quantité de la protéine HtrA1, dans des échantillons obtenus de sujets quelconques.

2. Méthode selon la revendication 1, où la protéine HtrA1 est constituée :
- à partir de la séquence d'acides aminés NP_002766.1 (SEQ. 1) ou
- à partir d'un fragment auto-protéolytique constitué de la forme 38kDa ou
- à partir de variantes de la protéine HtrA1 ayant une séquence d'acides aminés ayant au moins 80% d'identité d'acides aminés avec la séquence SEQ. 1 ou avec la séquence du respectif fragment auto-protéolytique constitué par la forme ou 38 kDa ou
- à partir d'une molécule qui comprend la séquence d'acides aminés SEQ. 1.

3. Méthode selon la revendication 2, dans laquelle la séquence d'acides aminés SEQ. 1 ou l'une de ses respectives variantes, ou la molécule de protéine qui comprend la séquence d'acides aminés SEQ. 1 sont immunoprécipitées de l'urine au moyen d'un procédé standard.

4. Méthode selon la revendication 3, dans laquelle les concentrations dans l'urine sont normalisées en fonction de la concentration de créatine.

5. Méthode selon la revendication 2, dans laquelle le fragment auto-protéolytique de la séquence d'acides aminés SEQ. 1 ou de ses variantes sont analysés directement dans l'extrait protéique des tissus de la vessie.

6. Méthode selon la revendication 5, dans laquelle la normalisation des extraits protéiques de la vessie est faite sur la base des valeurs de β-actine utilisée en tant que protéine saine (housekeeping).

7. Méthode selon l'une quelconque des revendications précédentes, dans laquelle les échantillons sont représentés par des fluides biologiques ou les tissus de la vessie du sujet.

8. Méthode selon la revendication 7, dans laquelle lesdits fluides biologiques comprennent l'urine.

9. Méthode selon l'une quelconque des revendications précédentes, dans laquelle on effectue une comparaison de la quantité de la protéine HtrA1 dans l'échantillon prélevé sur le sujet en cours d'examen avec celle mesurée dans l'échantillon de sujets considérés comme sains et dans laquelle une différence de ladite quantité entre les deux échantillons est associée à la présence d'un cancer urothélial de la vessie.

10. Méthode selon l'une des revendications de 2 à 8, dans laquelle une augmentation de la quantité de la protéine HtrA1 SEQ. 1 ou de ses variantes, ou de la molécule protéique comprenant la séquence d'acides aminés SEQ. 1, dans l'urine du sujet en cours d'examen par rapport à celle de sujets considérés sains est indicative d'un cancer urothélial de la vessie.

11. Méthode selon l'une des revendications de 2 à 8, dans laquelle une diminution de la quantité de fragment auto-protéolytique de la séquence d'acides aminés SEQ. 1 ou de ses variantes, selon les revendications 2, 5 et 6, dans le tissu du sujet en cours d'examen par rapport à celle de sujets considérés sains est indicative d'un cancer urothélial de la vessie.

12. Méthode pour surveiller l'efficacité du traitement du cancer urothélial de la vessie, dans laquelle la protéine HtrA1 est quantifiée selon la méthode des revendications 10 et 11, dans l'urine ou dans les tissus d'un patient.

13. Méthode d'imagerie du cancer de la vessie, dans laquelle on administre au patient un anticorps qui se lie spécifiquement à la protéine HtrA1 SEQ. 1 ou à son fragment auto-protéolytique ou à leurs respectives variantes, ou à la molécule protéique qui comprend la séquence d'acides aminés SEQ. 1, selon la revendication 2.

14. Méthode selon la revendication 13, où l'anticorps est conjugué à un marqueur relevable.
